Europäisches Patentamt

European Patent Office

Office européen des brevets

(11) Veröffentlichungsnummer: **0 381 991 B1**

# EUROPÄISCHE PATENTSCHRIFT

(45) Veröffentlichungstag der Patentschrift: **04.05.94**

(21) Anmeldenummer: **90101330.0**

(22) Anmeldetag: **23.01.90**

Die Akte enthält technische Angaben, die nach
dem Eingang der Anmeldung eingereicht wurden
und die nicht in dieser Patentschrift enthalten sind.

(51) Int. Cl.5: **G01N 33/569**, G01N 33/577,
G01N 33/543, G01N 33/532,
C12N 5/00

(54) Verfahren zur immunologischen Bestimmung von Bakterien der Gattung Aeromonas und der Spezies Vibrio cholerae.

(30) Priorität: **07.02.89 DE 3903582**

(43) Veröffentlichungstag der Anmeldung:
**16.08.90 Patentblatt 90/33**

(45) Bekanntmachung des Hinweises auf die
Patenterteilung:
**04.05.94 Patentblatt 94/18**

(84) Benannte Vertragsstaaten:
**AT BE CH DE DK ES FR GB GR IT LI LU NL SE**

(56) Entgegenhaltungen:

CHEMICAL ABSTRACTS, Band 108, Nr. 21, 23.
Mai 1988, Columbus, OH (US); J.MATSUDA et
al., Seite 573, Nr. 185273f&NUM;

CHEMICAL ABSTRACTS, Band 109, Nr. 9, 29.
August 1988, Columbus, OH (US);
J.MATSUDA et al., Seite 559, Nr.
71877w&NUM;

(73) Patentinhaber: **ORPEGEN MEDIZINISCH-MOLE-
KULARBIOLOGISCHE FORSCHUNGSGESELL-
SCHAFT m.b.H.
Czerny-Ring 22
D-69115 Heidelberg(DE)**

(72) Erfinder: **Carstens, Cornelia, Dipl.-Biol.
Robert Bollschweilerstrasse 12
D-6900 Heidelberg(DE)**
Erfinder: **Nader, Werner, Dipl.Biol. Dr.
Albert Lortzing-Strasse 5
D-6904 Eppelheim(DE)**
Erfinder: **Geiss, Heinrich-Konrad, Dr.
An der Neckarspitze 11
D-6900 Heidelberg(DE)**

CHEMICAL ABSTRACTS, Band 108, Nr. 13, 28. März 1988, Columbus, OH (US); J.MATSUDA et al., Seite 483, Nr. 110725w&NUM;

CHEMICAL ABSTRACTS, Band 104, Nr. 1, 06. Jänner 1986, Columbus, OH (US); T.HOLME et al., Seite 399, Nr. 4312s&NUM;

CHEMICAL ABSTRACTS, Band 99, Nr. 19, 07. November 1983, Columbus, OH (US); B.GUSTAFSSON et al., Seite 462, Nr. 156535h&NUM;

CHEMICAL ABSTRACTS, Band 106, Nr. 13, 30. März 1987, Columbus, OH (US); M.SAKAI et al., Seite 345, Nr. 98906&NUM;

(74) Vertreter: **Huber, Bernhard, Dipl.-Chem. et al Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**Beschreibung**

Die Erfindung betrifft ein Verfahren zum immunologischen Nachweis von Bakterien der Gattung Aeromonas oder der Spezies Vibrio cholerae durch Inkubation mit mindestens einem Rezeptor, der mit der nachzuweisenden Bakterienspezies bindefähig ist und seine Verwendung.

Die Gattung Aeromonas ist eine Gruppe von ubiquitär vorkommenden Bakterien, die als Erreger tierischer und menschlicher Infektionskrankheiten eine wichtige Rolle spielen. So wurden bis heute 500 Fälle von Infektionen mit Bakterien der Gattung Aeromonas in der Literatur beschrieben, die bei abwehrgeschwächten Patienten mit einer außerordentlich hohen Letalität einherging. Dies gilt insbesondere für schwerwiegende Infektionen wie Sepsis, Meningitis, Peritonitis, Pneumonie, Osteomyelitis und Myositis. Als Ursprung dieser Infektionen wird in der Regel kontaminiertes Wasser angenommen, wobei im Gegensatz zu anderen Erregern wie z.B. Salmonellen, die Aeromonaden nicht durch Fäkalien eingebracht werden, sondern in mit organischem Material belastetem Wasser ihren natürlichen Lebensraum haben. Vibrio cholerae ist ein mit Aeromonas eng verwandtes Bakterium, das als Erreger der epidemisch auftretenden Cholera bekannt ist und besonders in den Tropen als Erreger von Epidemien gefürchtet ist.

Auch in der Tierwelt verursachen Bakterien der Gattung Aeromonas Krankheiten, wobei von wirtschaftlicher Bedeutung die epidemisch auftretenden Infektionen von Lachs durch Aeromonas salmonicida in Zuchtanstalten sind.

Aeromonaden machen einen großen Anteil der Mikroflora in Abwasser aus, wobei ihr Anteil bis zu 30 % der auftretenden Bakterienmasse sein kann. Damit kommt ihnen eine Bedeutung als Indikatoren für Verunreinigungen von Trinkwasser zu.

Aeromonaden haben auch Bedeutung in der Klärtechnik beim Abbau organischer Substanzen. Es hat sich gezeigt, daß Aeromonaden in Kombination mit Acinetobacter calcoaceticus eine wichtige Rolle bei der biologischen Entphosphatierung spielt.

Der spezifische Nachweis dieser ubiquitär vorkommenden Bakterien ist daher sehr wichtig. Insbesondere ist es wichtig, die Gattung Aeromonas von den eng verwandten Enterobacteriaceae zu unterscheiden. Bisher erfolgte der Nachweis von Aeromonaden und Vibrio cholerae durch Wachstum auf Selektivmedien und die biochemische Charakterisierung der Isolate, wie sie beispielsweise mit verschiedenen Mikroidentifizier-Systemen erfolgt. Eine Differenzierung der eng miteinander verwandten Gattung Vibrio und Aeromonas ist dabei durch Einsatz eines Bakteriostatikums möglich, das selektiv das Wachstum von Vibrio hemmt. Dieses Verfahren ist jedoch aufwendig und liefert Ergebnisse erst nach einem relativ langen Zeitraum. Die schnelle Charakterisierung ist besonders bei schwerwiegenden Infektionskrankheiten von Bedeutung, um gezielt therapeutische Maßnahmen ergreifen zu können.

Holme und Gustafsson (Monoclonal Antibodies Bact. 1 (1985), 167-189) und Matsuda et al. (CA 108:185273f) beschreiben monoklonale Antikörper gegen das O-Antigen von Vibrio cholerae, die eine Unterscheidung einzelner Subspezies des Bakteriums ermöglichen Ein Antikörper, der einen gemeinsamen Nachweis aller Subspezies ermöglicht, wird nicht offenbart.

Popoff (in Bergey's Manual of Systematic Bacteriology 1, Ed. N.R. Krieg und J.G. Holt, Williams & Wilkins, Baltimore, 1984, Seiten 545-546) beschreibt zwölf verschiedene O-Antigene bei einer einzigen Aeromonas-Spezies, die eine exakte Serotypisierung von Subspezies erlauben. Ein monoklonaler Antikörper, der einen gemeinsamen Nachweis von Bakterien des Genus Aeromonas ermöglicht, wird nicht offenbart.

Es bar daher Aufgabe der vorliegenden Erfindung, ein Verfahren zum Nachweis von Bakterien der Gattung Aeromonas oder der Spezies Vibrio cholerae zur Verfügung zu stellen, das bei hoher Spezifität die schnelle und einfache Identifizierung dieser Bakterien erlaubt und exakte reproduzierbare Ergebnisse liefert.

Diese Aufgabe wird gelöst durch ein Verfahren zum immunologischen Nachweis von Bakterien der Gattung Aeromonas oder der Spezies Vibrio cholerae durch Inkubation mit mindestens einem Rezeptor, der mit dem nachzuweisenden Bakterium bindefähig ist, welches dadurch gekennzeichnet ist, daß man als Rezeptor einen monoklonalen Antikörper, der mit einem allen Bakterien der Spezies Aeromonas und Vibrio cholerae gemeinsamen O-Antigen bindefähig ist, in immobilisierter, immobilisierbarer oder markierter Form verwendet.

Der Nachweis von Bakterien der Gattung Aeromonas oder der Spezies Vibrio cholerae kann mit dem erfindungsgemäßen Verfahren schnell und einfach durchgeführt werden. Bakterien der nah verwandten Gattung Enterobacteriaceae reagieren nicht, während praktisch alle bekannten Aeromonasarten eine positive Reaktion zeigen, ebenso wie einige Stämme von Vibrio cholerae.

Dies ist möglich durch die Anwendung immunologischer Methoden, d.h. die Mikroorganismen werden über die Bindung spezifischer Antikörper mit Oberflächenantigenen der Bakterien erkannt. Als spezifische Antikörper werden monoklonale Antikörper in immobilisierter, immobilisierbarer oder markierter Form verwendet, die ausschließlich an allen Aeromonas spp. gemeinsamen Zelloberflächenstrukturen binden. Weiterhin kommt es zur Reaktion mit einem Teil der Vertreter der Spezies Vibrio cholerae. Andere Arten der Familie Vibrioaceae und Vertreter der Familien Enterobacteriaceae, Pseudomonaceae, Neisseriaceae, Micrococcaceae und Streptococcaceae (Tab. 1) zeigten keine Affinität zum dargestellten Antikörper.

Wesentliches Merkmal des erfindungsgemäßen Verfahrens ist die Verwendung eines für Bakterien der Gattung Aeromonas und der Spezies Vibrio cholerae spezifischen monoklonalen Antikörpers. Es wurde gefunden, daß alle bekannten Aeromonas-Stämme sowie die meisten Vibrio cholerae-Stämme ein gemeinsames Antigen aufweisen, das als O-Antigen identifiziert werden konnte. Ein für dieses Oberflächen-Antigen spezifischer Antikörper kann erhalten werden durch Immunisierung eines geeigneten Organismus, Fusion gebildeter B-Lymphozyten mit Myelomzellen und Screening nach Zellinien, die Antikörper produzieren, die mit Aeromonaden und Vibrio cholerae spezifisch binden, nicht aber mit nah verwandten anderen Bakterien wie z.B. Enterobacteriaceae.

Der erfindungsgemäße Nachweis erfolgt im Rahmen eines immunologischen Bestimmungsverfahrens, das quantitativ oder qualitativ erfolgen kann. Immunologische Nachweisverfahren sind in vielen Varianten bekannt und alle gängigen Varianten sind für die Durchführung des erfindungsgemäßen Verfahrens geeignet.

Zur quantitativen Bestimmung können z.B. Verfahren nach dem Prinzip des Immunoassay wie ELISA, IEMA, IRMA, IFMA usw. verwendet werden. Dazu wird die Probe mit einem, zwei oder auch mehreren Rezeptoren inkubiert, wobei ein Rezeptor immobilisiert, immobilisierbar und/oder markiert ist. Die Auswertung der Reaktion erfolgt über Agglutination oder Markierung. Als Markierung sind radioaktive Isotope, Enzyme, fluoreszierende, chemilumineszierende oder Farbstoff bildende Substanzen geeignet. Verfahren zur Markierung ebenso wie zum Nachweis der Markierung sind dem Fachmann bekannt und brauchen hier nicht näher erläutert werden.

Die im folgenden beschriebenen Varianten zur Durchführung des erfindungsgemäßen Verfahrens sind besonders geeignet zum Nachweis des Vorkommens der Aeromonaden bzw. Vibrio cholerae in Flüssigkeiten. Dazu wird bevorzugt ein Immunoassay verwendet, wie z.B. der als ELISA bekannte Test. In einer Ausführungsform werden die Bakterien der zu testenden Probe an eine Mikrotiterplatte durch Auftrocknen fest gebunden und dadurch immobilisierbar gemacht. Die Festphase wird dann mit einem hochspezifischen Antikörper inkubiert. Bei der Inkubation bindet der monoklonale Antikörper mit den immobilisierten oder immobilisierbaren Aeromonaden, die in der Probelösung vorliegen. Die Auswertung dieser immunologischen Reaktion erfolgt über eine Markierung. Dabei kann entweder der Antikörper selbst in an sich bekannter Weise markiert sein, im Falle des ELISA's z.B. mit einem Enzym. In einer anderen Variante ist der monoklonale Antikörper unmarkiert und es wird ein weiterer Rezeptor verwendet, der z.B. ein gegen den konstanten Teil des monoklonalen Antikörpers gerichteter markierter Antikörper sein kann. Die Auswertung der Markierung erfolgt in an sich bekannter Weise. Weitere bekannte Varianten dieses Verfahrensprinzips sind ebenfalls geeignet.

Eine weitere bevorzugte Ausführungsform des erfindungsgemäßen Verfahrens besteht darin, daß man die Probe, die auf Aeromonaden untersucht werden soll, mit dem monoklonalen Antikörper inkubiert und anschließend einen gegen den konstanten Teil des Antikörpers gerichteten, mit einer fluoreszierenden oder chemilumineszierenden Substanz markierten Antikörper zugibt. Auf diese Weise werden die Aeromonaden durch ihre Fluoreszenz sichtbar, so daß sie qualitativ im Fluoreszenzmikroskop und quantitativ durch Auswertung in einem Durchflußzytometer nachgewiesen werden können. Zum Nachweis können dabei die Bakterien durch Hitze an einen Objektträger mit 0,5 bis 3 % Formaldehyd oder 70 % Alkohol fixiert werden.

In einer weiteren Variante werden Bakterien der Spezies Staphylococcus aureus, auf deren Oberfläche sich Protein A befindet bzw. daraus isoliertes Protein A mit den erfindungsgemäßen spezifischen monoklonalen Antikörpern inkubiert, wobei der Antikörper mit seinem konstanten Teil an das Protein A bindet. Anschließend wird die Probelösung zugegeben. Bei Bindung der Aeromonaden verklumpen die Bakterien miteinander, so daß eine Trübung auftritt. Diese Trübung kann dann in üblicher Weise ausgewertet werden. Da etwa ein Drittel der bekannten Aeromonas-Stämme in physiologischer Kochsalzlösung von selbst agglutiniert, ist es bevorzugt, die Selbstverklumpung durch Zugabe von Formaldehyd zu hemmen.

Eine Zellinie, die für das erfindungsgemäße Verfahren besonders geeignete Antikörper liefert und daher auch Gegenstand der Erfindung ist, ist die bei ECACC mit der Nummer 89011701-Aero/OH1 hinterlegte Zellinie.

Das erfindungsgemäße Verfahren eignet sich zur immunologischen Bestimmung von Aeromonaden und Vibrio cholerae in vielen Bereichen. Einerseits dient es zum Nachweis von Erkrankungen in der klinischen und tiermedizinischen Diagnostik und andererseits dient es als Parameter für die Untersuchung von Wasser und Abwasser.

4

In der klinischen Diagnostik wird in einem immunologischen Verfahren das Vorhandensein von Bakterien der Gattung Aeromonas oder der Spezies Vibrio cholerae in Körperflüssigkeiten, vor allem Blut, Urin oder Stuhl bestimmt. Bei unspezifischen Infektionen wie beispielsweise Meningitis oder Sepsis ist es sehr wesentlich festzustellen, welches Bakterium die Infektion verursacht, um dieses dann gezielt therapeutisch bekämpfen zu können. Da sich das Krankheitsbild der Cholera signifikant von dem eines durch Aeromonaden verursachten Durchfalls unterscheidet, ist eine Verwechslung dieser beiden Stämme nicht möglich.

In der tiermedizinischen Diagnostik kann das erfindungsgemäße Verfahren zum Nachweis von durch Aeromonaden verursachten Krankheiten verwendet werden. Aeromonasinfektionen treten vor allem bei wechselwarmen Tieren auf. Insbesondere kann das Verfahren zum Nachweis von Infektionen mit Aeromonas salmonicida in Fischzuchtanstalten verwendet werden.

Da Aeromonas in einem hohen Prozentsatz im Abwasser vorkommt, kann es als Kontrollbakterium in der Untersuchung von Brauch- und Trinkwässern verwendet werden. Wenn in einem Trink- oder Brauchwasser Aeromonaden nachgewiesen werden können, kann auf eine Verschmutzung durch Abwasser geschlossen werden und außerdem gibt der Nachweis von Aeromonaden Hinweise auf den Ursprung der Verschmutzung. Das erfindungsgemäße Verfahren wird daher bevorzugt auch in der Wasseruntersuchung eingesetzt.

Weiterhin kann das erfindungsgemäße Verfahren vorteilhaft zur Überwachung der Belebtbecken und Faultürme in Klärwerken verwendet werden. Aeromonas ist ein an der biologischen Entphosphatierung von Abwässern beteiligter Mikroorganismus, dessen Nachweis Rückschlüsse auf die Leistungsfähigkeit des Klärwerks zuläßt.

Das Verfahren der vorliegenden Erfindung erlaubt es, schnell und einfach Aeromonaden nachzuweisen und kann daher vorteilhaft in vielen Gebieten verwendet werden.

Die Erfindung wird durch die folgenden Figuren und Beispiele erläutert.

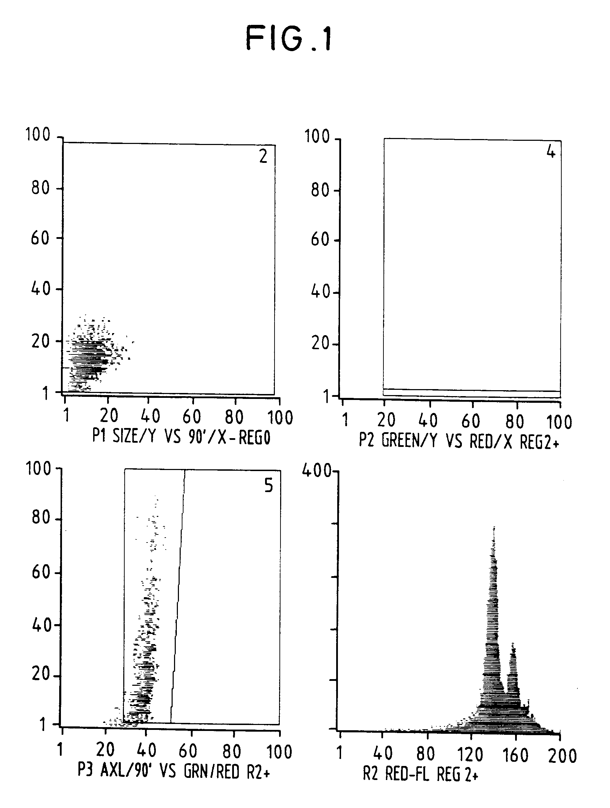

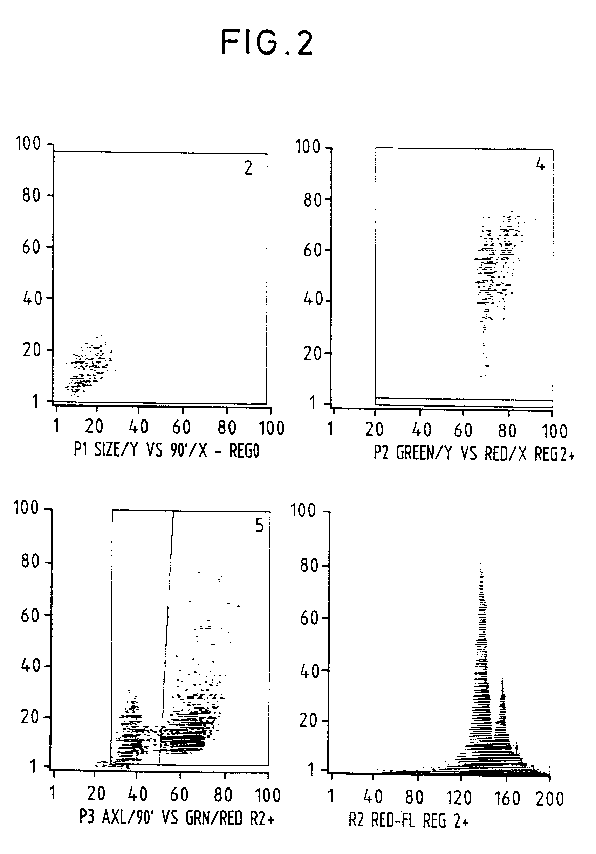

Fig. 1 und 2    zeigen Cytogramme der Rot- gegen die Grünfluoreszenz, die mit einem Durchflußcytometer erhalten wurden.

## Beispiel 1

Es wurde eine Zellinie gewonnen, die spezifisch mit Aeromonaden bindefähige monoklonale Antikörper produziert. Dazu wurde Mäusen der Zuchtlinie Balb/c in Abständen von je 5 Tagen insgesamt 13mal $10^7$ Zellen von Bakterien der Spezies Aeromonas hydrophila subspecies punctata injiziert. Dieser Stamm war aus dem Abwasser der Stadt Heidelberg isoliert worden und vor der Immunisierung mit 1 % Formalin abgetötet worden. Die erste Immunisierung erfolgte subcutan, die weiteren 10 Immunisierungen intraperitoneal und die letzten zwei Immunisierungen intravenös.

Nach Abtötung der Tiere und Entnahme der Milz wurden die Milzzellen mit Maus-Myelomzellen der permanenten Linie P 3 x 63 Ag•8U.1 nach der Polyethylenglykol-Methode fusioniert.

Die fusionierten Zellen wurden auf HAT(Hypoxanthin-Aminopterin-Thymidin)-Medium selektioniert, weiter in HT-Medium (Hypoxanthin-Thymidin) kloniert und schließlich in normales Zellkulturmedium (RPMI 1640) überführt. Es wurden die in Peters et al., "Monoklonale Antikörper: Herstellung und Charakterisierung", Springer-Verlag, Berlin (1985) beschriebenen Medien verwendet. Der Test der Zellüberstände auf spezifische monoklonale Antikörper erfolgte auf mit Bakterien beschichteten Mikrotiterplatten über den ELISA-Test. Das Screening wurde mit folgenden Stämmen durchgeführt:

a) Aeromonas hydrophila hydrophila API 20E Code: 7046527

b) Aeromonas hydrophila punctata API 20E Code: 3046127

c) Gemisch aus 11 verschiedenen Aeromonas-Isolaten aus dem Abwasser der Stadt Heidelberg.

Es wurde eine Zellinie gewonnen, die Antikörper produziert, die mit allen zum Screening eingesetzten Aeromonaden reagierte. Diese Zellinie wurde unter der Nummer ECACC 89011701-Aero/OH1 bei ECACC hinterlegt.

Die von dieser Zellinie produzierten Antikörper wurden dann auf ihre Affinität zu 146 verschiedenen Bakterien-Stämmen aus 8 verschiedenen Familien und Gruppen getestet. Die Ergebnisse sind der Tabelle 1 von Beispiel 2 zu entnehmen.

## Beispiel 2

Aeromonaden und Proben von Klärschlamm und Abwasser, die Aeromonaden enthielten, wurden an Mikrotiterplatten durch Auftrocknen gebunden. Es wurde gezeigt, daß Aeromonaden quantitativ an Mikrotiterplatten des Fabrikats "Nunc-Immuno-Plate Maxi Sorp F 96" (Nune A/S, Roskilde, Dänemark) binden und sich auch durch längeres Waschen mit PBS (Phosphate-Buffered-Saline) nicht ablösen lassen. Die Platten wurden anschließend mit 1%iger Rinderserumproteinlösung mehrere Stunden bei 4°C inkubiert und dann mit einer 0,00023%igen Lösung des monoklonalen Antikörpers ECACC 89011701 45 Minuten inkubiert. Die Platten wurden gewaschen und dann mit einem Peroxidase-konjugierten Ziegen-Antikörper gegen die Immunglobuline G der Maus inkubiert. Nach mehreren Waschvorgängen wurde dann Peroxidase durch Reaktion mit ortho-Phenylendiamin und $H_2O_2$ kolorimetrisch bei 492 nm nachgewiesen. Es wurden verschiedene Stämme getestet und die Ergebnisse sind der folgenden Tabelle 1 zu entnehmen.

## Tabelle 1

Affinität des monoklonalen Antikörpers gegen verschiedene Bakterienstämme, bestimmt im ELISA-Test

| Familie | Spezies | Anzahl der getesteten Stämme | Affinität |
|---|---|---|---|
| Enterobacteriaceae | Escherichia coli | 4 | — |
| | Klebsiella pneumoniae | 2 | — |
| | Klebsiella oxitoka | 1 | — |
| | Proteus vulgaris | 2 | — |
| | Salmonella spp. | 4 | — |
| | Shigella boydii | 1 | — |
| | Kluyvera spp. | 2 | — |
| | Shigella sonnei | 2 | — |
| | Shigella flexneri | 1 | — |
| Pseudomonaceae | Pseudomonas aeruginosa | 1 | — |
| | Ps. fluorescens | 1 | — |
| | Ps. maltophilia | 1 | — |
| | Ps. oryzihabitans | 1 | — |
| | Ps. putida | 1 | — |
| Neisseriaceae | Acinetobacter calcoaceticus | 1 | — |
| | Acinetobacter calcoac. wolffii | 3 | |
| Micrococcaceae | Micrococcus spp. | 2 | — |
| | Staphylococcus aureus | 3 | — |
| | Staphylococcus epidermidis | 1 | |
| Streptococcaceae | Streptococcus faecalis | 1 | — |

EP 0 381 991 B1

T a b e l l e  1
(Fortsetzung)

| Familie oder Gruppe | Spezies | | Anzahl der getesteten Stämme | Affinität |
|---|---|---|---|---|
| Nocardiaforme | Nocardia spp. | | 1 | - |
| Sphaerotilus-Leptothrix | Sphaerotilus natans | | 1 | - |
| Vibrionaceae | Plesiomonas spp. | | 1 | - |
| | Plesiomonas shigelloides | | 2 | - |
| | Vibrio metschnikovii | | 1 | - |
| | Vibrio parahaemolyticus | | 5 | - |
| | Vibrio alginolyticus | | 3 | - |
| | Vibrio cholerae | NAG | 3 | - |
| | Vibrio cholerae | NAG | 2 | + |
| | Vibrio cholerae | Inaber EL TOR | 3 | + |
| | Vibrio cholerae | Ogawa EL TOR | 1 | + |
| | Vibrio cholerae | Hikojima | 1 | + |
| | Aeromonas hydrophila | hydrophila | 14 | + |
| | Aeromonas hydrophila | punctata | 8 | + |
| | Aeromonas caviae | | 15 | + |
| | Aeromonas hydrophila | (aus Stuhl) | 17 | + |
| | Aeromonas sobria | | 4 | + |
| | Aeromonas salmonicida | salmonicida | 2 | + |
| | Aeromonas salmonicida | achromogenes | 1 | + |
| | Aeromonas salmonicida | masoncida | 1 | + |
| | Aeromonas salmonicida | schleie | 1 | + |
| | Aeromonas salmonicida | | 5 | + |
| | Aeromonas spp. | (Isolate aus Stuhlproben) | 19 | + |

**Beispiel 3**

Es wurden in einer Probelösung Aeromonaden über Immunfluoreszenz nachgewiesen. Dazu wurde die Probelösung mit einer die oben beschriebenen monoklonalen Antikörper enthaltenden Lösung eine Stunde

8

inkubiert. Die Probe wurde anschließend gewaschen und dann mit einem mit Fluorescein-thiocyanat konjugierten Ziegen-Antikörper gegen Immunglobulin G der Maus inkubiert. Dabei bildeten sich Komplexe aus Aeromonaden, dem monoklonalen Antikörper und dem fluoreszenzmarkierten Antikörper. Die Fluoreszenz konnte dann unter dem Fluoreszenzmikroskop betrachtet werden und im Durchflußcytometer nachgewiesen werden. Die Ergebnisse sind den Cytogrammen in Abbildung 1 zu entnehmen.

**Beispiel 4**

Bakterien der Spezies Staphylococcus aureus wurden mit dem oben beschriebenen monoklonalen Antikörper gegen Aeromonas beladen. Der Antikörper kuppelte dabei mit seinem konstanten Teil an das Protein A auf der Oberfläche dieser Bakterien. Bei Zugabe der so derivatisierten Staphylococcen zu Suspensionen, die Aeromonaden enthalten, trat durch Bindung der Aeromonaden an die Antikörper eine Verklumpung auf, die zu einer mit dem bloßen Auge sichtbaren Präzipitation führte. Für diesen Test wurden zwei Aeromonas Salmonicida-Stämme und sieben verschiedene Aeromonaden aus Stuhl eingesetzt.

**Patentansprüche**

1.  Verfahren zum immunologischen Nachweis von Bakterien der Gattung Aeromonas oder der Spezies Vibrio cholerae durch Inkubation mit mindestens einem Rezeptor, der mit dem nachzuweisenden Bakterium bindefähig ist, **dadurch gekennzeichnet,** daß man als Rezeptor einen monoklonalen Antikörper, der mit einem allen Bakterien der Gattung Aeromonas und der Spezies Vibrio cholerae gemeinsamen O-Antigen bindefähig ist in immobilisierter, immobilisierbarer oder markierter Form, verwendet.

2.  Verfahren nach Anspruch 1, **dadurch gekennzeichnet,** daß man als monoklonalen Antikörper einen von der Zellinie ECACC 89011701-Aero/OH1 produzierten Antikörper verwendet.

3.  Verwendung des Verfahrens nach Anspruch 1 oder 2 zum Nachweis einer Aeromonas-Infektion in der klinischen Diagnostik.

4.  Verwendung des Verfahrens nach Anspruch 1 oder 2 zum Nachweis einer Aeromonas-Infektion in der Tiermedizin.

5.  Verwendung des Verfahrens nach Anspruch 1 oder 2 zur Wasseruntersuchung.

6.  Verwendung des Verfahrens nach Anspruch 1 oder 2 zur Kontrolle von Klärwerken.

7.  Zellinie ECACC 89011701-Aero/OH1.

**Claims**

1.  Process for the immunological detection of bacteria of the genus Aeromonas or of the species Vibrio cholerae by incubation with at least one receptor which is bindable with the bacterium to be detected, characterised in that, as receptor, one uses a monoclonal antibody which is bindable with an O-antigen common to all bacteria of the genus Aeromonas and the species Vibrio cholerae in immobilised, immobilisable or labelled form.

2.  Process according to claim 1, characterised in that, as monoclonal antibody, one uses an antibody produced by the cell line ECACC 89011701-Aero/OH1.

3.  Use of the process according to claim 1 or 2 for the detection of an Aeromonas infection in clinical diagnosis.

4.  Use of the process according to claim 1 or 2 for the detection of an Aeromonas infection in veterinary medicine.

5.  Use of the process according to claim 1 or 2 for water investigation.

**6.**   Use of the process according to claim 1 or 2 for the monitoring of clarification plant.

**7.**   Cell line ECACC 89011701-Aero/OH1.

**Revendications**

**1.**   Procédé pour l'identification immunologique de bactéries du genre Aeromonas ou de l'espèce Vibrio cholerae par incubation avec au moins un récepteur qui est capable de se fixer sur la bactérie à identifier, caractérisé en ce qu'on utilise comme récepteur un anticorps monoclonal qui est capable de se fixer sur un antigène O commun à toutes les bactéries du genre Aeromonas et de l'espèce Vibrio cholarae sous la forme immobilisée, immobilisable ou marquée.

**2.**   Procédé selon la revendication 1, caractérisé en ce qu'on utilise comme anticorps monoclonal un anticorps produit par la lignée de cellules ECACC 89011701-Aero/OH1.

**3.**   Utilisation du procédé selon la revendication 1 ou 2 pour la détection d'une infection par Aeromonas dans le diagnostic clinique.

**4.**   Utilisation du procédé selon la revendication 1 ou 2 pour la détection d'une infection par Aeromonas en médecine vétérinaire.

**5.**   Utilisation du procédé selon la revendication 1 ou 2 pour l'examen de l'eau.

**6.**   Utilisation du procédé selon la revendication 1 ou 2 pour le contrôle des stations d'épuration.

**7.**   Lignée de cellules ECACC 89011701-Aero/OH1.

# FIG.1

# FIG.2

P1 SIZE/Y VS 90'/X - REG0

P2 GREEN/Y VS RED/X REG2+

P3 AXL/90' VS GRN/RED R2+

R2 RED-FL REG 2+